(19) Europäisches Patentamt
European Patent Office
Office européen des brevets

(11) Publication number: **0 192 935 B1**

(12) **EUROPEAN PATENT SPECIFICATION**

(45) Date of publication of patent specification: **27.02.91**

(51) Int. Cl.5: **C07D 401/04**, A61K 31/415

(21) Application number: **86100347.3**

(22) Date of filing: **13.01.86**

# (54) 3-(piperidinyl)-1H-indazoles a process for their preparation and their use as medicaments.

(30) Priority: **23.01.85 US 694198**

(43) Date of publication of application:
**03.09.86 Bulletin 86/36**

(45) Publication of the grant of the patent:
**27.02.91 Bulletin 91/09**

(84) Designated Contracting States:
**AT BE CH DE FR GB IT LI LU NL SE**

(56) References cited:
**EP-A- 0 135 781**
**EP-A-12 716 7**
**US-A- 4 355 037**

(73) Proprietor: **HOECHST-ROUSSEL PHARMACEUTICALS INCORPORATED**
**Route 202-206 North**
**Somerville New Jersey 08876(US)**

(72) Inventor: **Strupczewski, Joseph T.**
**4 Stewart Lane**
**Flemington, N.J. 08822(US)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80(DE)**

**EP 0 192 935 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel 3-(piperidinyl)-1H-indazoles of the formula I

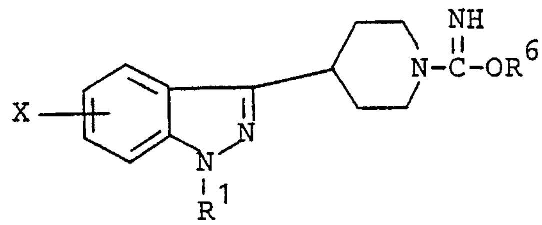

wherein

R¹ is hydrogen, $C_1$-$C_7$-alkyl, di-$C_1$-$C_7$-alkylamino-$C_1$-$C_7$-alkyl, hydroxy-$C_1$-$C_7$-alkyl, $C_3$-$C_7$-cycloalkyl-$C_1$-$C_7$-alkyl, a radical of the formula

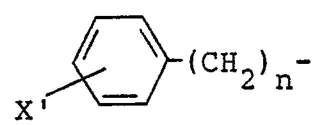

where n is an integer of 0 to 4 and X' is hydrogen, halogen or $CF_3$,

R⁶ is $C_1$-$C_7$-alkyl, $C_3$-$C_7$-cycloalkyl-$C_1$-$C_7$-alkyl or $C_1$-$C_7$-alkyl substituted with amino, $C_1$-$C_7$-alkylamino or di-$C_1$-$C_7$-alkylamino,

X is hydrogen or halogen,

the optical antipodes or the pharmaceutically acceptable salts thereof, which are useful for treating psychoses, treating depression, treating and alleviating pain and treating hypertension, alone or in combination with inert psychoses - treating, depression treating, pain - alleviating and hypertension alleviating adjuvants.

U.S.-A-4,355,037 relates to 3-(4-piperidyl)-1,2-benzisoxazoles which exhibit analgesic activity. EP-A-0 127 167 relates to 3-(4-piperidinyl)-1,2-benzisothiazoles which show antipsychotic and analgesic activity.

As used through the specification and appended claims, the term "alkyl" refers to a straight or branched chain hydrocarbon radical containing no unsaturation and having 1 to 7 carbon atoms such as methyl, ethyl, 1-propyl, 2-propyl, 1-butyl, 1-pentyl, 2-pentyl, 3-hexyl, 4-heptyl; the term "alkenyl" refers to a straight or branched chain hydrocarbon radical having one olefinic bond and containing 3 to 7 carbon atoms such as 2-propenyl, 2-butenyl, 3-pentenyl, 3-hexenyl, 3-heptenyl; the term "cycloalkyl" refers to a saturated hydrocarbon group possessing at least one carbocyclic ring, the ring containing from 3 to 7 carbon atoms such as cyclopropyl, cyclobutyl, cyclopentyl, cyclohexyl, cycloheptyl; the term "alkanol" refers to a compound formed by a combination of an alkyl group and a hydroxy radical. Examples of alkanols are methanol, ethanol, 1- and 2-propanol, 1,2-dimethylethanol, hexanol. The term "alkoxy" refers to a radical formed by removal of the hydrogen atom from the hydroxy function of an alkanol. Examples of alkanols are methoxy, ethoxy, 1- and 2-propoxy, 1,2-dimethylethoxy, hexoxy. The term "alkanoic acid" refers to a compound formed by combination of a carboxyl group with a hydrogen atom or alkyl group. Examples of alkanoic acids are formic acid, acetic acid, propanoic acid, 2,2-dimethylacetic acid, hexanoic acid; the term "halogen" refers to a member of the family consisting of fluorine, chlorine, bromine or iodine. The term "alkanoyl" refers to the radical formed by removal of the hydroxyl function from an alkanoic acid. Examples of alkanoyl groups are formyl, acetyl, propionyl, 2,2-dimethylacetyl, hexanoyl. The term "alkylene" refers to a bivalent radical of the lower branched or unbranched group it is derived from having valence bonds from two terminal carbons thereof, e.g. ethylene ( $-CH_2-CH_2-$) , propylene ( $-CH_2CH_2CH_2-$), isopropylene ( $CH_2-CH-CH_2-$).

The compounds of the present invention which lack an element of symmetry exist as optical antipodes and as the racemic forms thereof. The optical antipode may be prepared from the corresponding racemic forms by standard optical resolution techniques, involving, for example, the separation of diastereomeric salts of those instant compounds characterized by the presence of a basic amino group and an optically active acid, or by the synthesis from optically active precursors.

The present invention comprehends all optical isomers and racemic forms thereof of the compounds disclosed and claimed herein. The formulas of the compounds shown herein are intended to encompass all optical isomers of the compounds so depicted.

The preparation of compounds of the formula II,

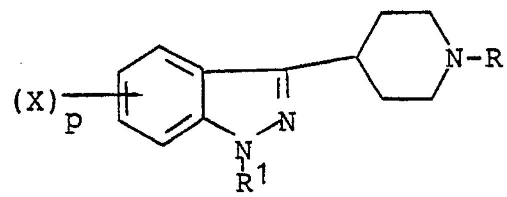

where $R^1$ is as defined above and R' is hydrogen or cyano, is described in published European Patent Application EP-A1-0 135 781.

1H-Indazoles substituted at the 1-position by a $C_1$-$C_7$-alkyl group, i.e. compounds of formula II wherein $R^1$ is $C_1$-$C_7$-alkyl, can also be prepared by reducing a 1-($C_1$-$C_7$)-alkanoyl-or 1-($C_1$-$C_7$)-alkoxycarbonyl-3-(piperidinyl)-1H-indazole by an alkali metal aluminum hydride such as, for example, lithium aluminum hydride, in an ethereal solvent such as tetrahydrofuran, at the reflux temperature of the reaction medium.

1H-Indazoles of formula I wherein $R^1$ and X are as defined above and $R^6$ is as previously defined, are prepared from 1H-indazoles of formula II where R' is cyano, or from 1-substituted 1H-indazoles of formula III

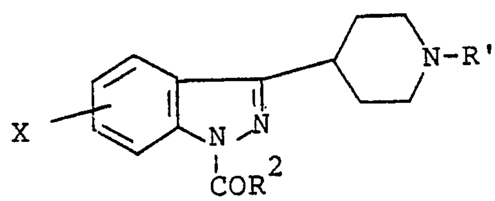

where R' is cyano and $R^2$ and X are as above, by reaction with an alcohol of the formula $R^6OH$ , typically in the presence of an alkali metal cyanide such as potassium cyanide. Ordinarily the reaction is conducted in the presence of alkali metal cyanide such as potassium cyanide and an excess amount of the alcohol $R^6OH$ , which also works as a reaction medium. A typical reaction condition is refluxing the reaction mixture for several hours and then further continuing the reaction at ambient temperature for 10 - 20 hours. In an alternative procedure, the 1H-indazole, above, where R' is cyano, is reacted with an alcohol of the formula $R^6OH$ in the presence of (usually only catalytic amount) an alkalic metal alkoxide of formula $MOR^6$ where M is an alkali metal, preferably sodium. Usually sodium metal is added to an excess amount of an alcohol of formula $R^6OH$ to form the sodium alkoxide of formula $NaOR^6$. Thereafter the cyano substituted 1H-indazole II is added to the mixture and if necessary the mixture is heated slightly until a uniform solution is formed.

To prepare 1H-indazoles substituted at the 1-position, i.e. compounds of formula I wherein $R^1$ is $C_1$-$C_7$-alkyl, $C_3$-$C_7$-cycloalkyl-$C_1$-$C_7$-alkyl or di-$C_1$-$C_7$-alkylamino-$C_1$-$C_7$-alkyl, or a radical of the formula

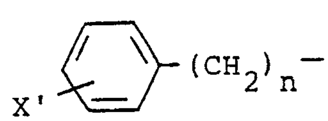

where X' is as defined above,
a 1-unsubstituted 1H-indazole of the formula I is treated, respectively, with an $C_1$-$C_7$-alkyl, $C_3$-$C_7$-cycloalkyl-$C_1$-$C_7$-alkyl or di-$C_1$-$C_7$-alkylamino-$C_1$-$C_7$-alkyl halide, i.e. an iodide, bromide or chloride, or with a compound of the formula

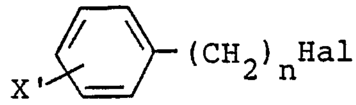

wherein X' is as defined above and Hal is iodo, bromo or chloro, in the presence of an alkali metal hydride suspended in a polar aprotic solvent. Among alkali metal hydrides, there may be mentioned lithium hydride, potassium hydride and sodium hydride. Sodium hydride is preferred. Among polar aprotic solvents, there may be mentioned dimethylformamide, dimethylacetamide and hexamethylphosphoramide. Dimethylformamide is preferred. While the alkylation normally proceeds readily at ambient temperature, the reaction may be conducted at an elevated temperature of about 50° to about 100° C to facilitate the conversion.

The 3-(piperidinyl)-1H-indazoles of the present invention are useful for treating psychoses by virtue of their ability to elicit an antipsychotic response in mammals.

Antipsychotic activity is determined in the climbing mice assay by methods similar to those described by P. Protais et al., Psychopharmacol., 50, 1 (1976) and B. Costall, Eur.J. Pharmacol., 50 , 39 (1978).

Antipsychotic response is achieved when the present 3-(piperidinyl)-1H-indazoles are administered to a subject requiring such treatment as an effective oral, parenteral or intravenous dose of from 0.01 to 50 mg/kg of body weight per day. A particularly preferred effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only.

The 3-(piperidinyl)-1H-indazoles of the present invention are also useful as analgetics due to their ability to alleviate pain in mammals. The analgetic utility is demonstrated in the phenyl-p-quinone writhing assay in mice, a standard assay for analgesia [Proc. Soc. Exptl. Biol. Med., 95 , 729 (1957)].

Analgesia production is achieved when the present 3-(piperidinyl)-1H-indazoles are administered to a subject requiring such treatment as an effective oral, parenteral or intravenous dose of from 0-01 to 100 mg/kg of body weight per day. A particularly effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only.

The 3-(piperidinyl)-1H indazoles of the present invention are also useful as antidepressants by virtue of their ability to elicit an antidepressant response in mammals. The antidepressant activity is demonstrated in the tetrabenazine induced ptosis assay in mice [International Journal of Neuropharmacology, 8 , 72 (1969)], a standard assay for antidepressant activity.

Antidepressant response is achieved when the present 3-(piperidinyl) -1H-indazoles are administered to a subject requiring such treatment as an effective oral, parenteral or intravenous dose of from 0.01 to 50 mg/kg of body weight per day. A particularly preferred effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only.

The compounds of the invention are also useful as antihypertensive agents due to their ability to depress blood pressure in mammals. Antihypertensive activity is measured in the spontaneous hypertensive rat by the indirect tail cuff method described in "Methods in Pharmacology," A. Schwartz, Ed., Vol. 1, Appleton-Century Crofts, New York, 1971, p. 135. In this procedure a group of five animals are treated orally for three days with the test compound in relation to the control group of the same number. The drop in blood pressure is measured on the third day following administration. The antihypertensive activities of some of the compounds, expressed as mm decrease in mean arterial blood pressure are given below:

| Compound | Dose (mg/kg of body weight, parenteral) | Decrease in Blood Pressure (mm Hg) |
|---|---|---|
| 4-(1H-indazol-3-yl)piperidine-1-carboximidic acid methyl ester | 3 | 47 |
| 4-(6-fluoro-1H-indazol-3-yl) piperidine-1-carboximidic acid methyl ester | 30 | 88 |
| guanethidine | 50 | 20 |

Blood pressure reduction is achieved when the compounds of the invention are administered to a subject requiring such treatment at an effective oral, parenteral or intravenous does of from 0.1 to 50 mg/kg of body weight per day. A preferred effective dose within this range is from about 0.1 to 5 mg/kg of body weight per day. A particularly preferred effective amount is about 1 mg/kg of body weight per day. It is to be understood, however, that for any particular subject, specific dosage regimens should be adjusted according to the individual need and the professional judgment of the person administering or supervising the administration of the compounds of the invention. It is to be further understood that the dosages set forth herein are examples only.

Effective amounts of the present invention may be administered to a subject by any one of various methods, for example, orally as in capsules or tablets, parenterally in the form of sterile solutions or suspensions, and in some cases intravenously in the form of sterile solutions. The 3-(piperidinyl)-1H-indazoles of the present invention, while effective themselves, may be formulated and administered in the form of their pharmaceutically acceptable addition salts for purposes of stability, convenience or crystallization, increased solubility.

Preferred pharmaceutically acceptable addition salts include salts of mineral acids, for example, hydrochloric acid, sulfuric acid or nitric acid, salts of monobasic carboxylic acids such as, for example, acetic acid or propionic acid, salts of dibasic carboxylic acids such as, for example, maleic acid or fumaric acid, and salts of tribasic carboxylic acids such as, for example, carboxysuccinic acid or citric acid.

Effective quantities of the compounds of the invention may be administered orally, for example, with an inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers or chewing gums. These preparations should contain at least 0.5% of active compound, but may be varied depending upon the particular form and may conveniently be between 4% to about 70% of the weight of the unit. The amount of active compound in such composition is such that a suitable dosage will be obtained. Preferred compositions and preparations according to the present invention are prepared so that an oral dosage unit form contains between 1.0-300 milligrams of the active compound.

The tablets, pills, capsules or troches may also contain the following ingredients: a binder such as microcrystalline cellulose, gum tragancanth or gelatin; an excipient such as starch or lactose, a disintegrating agent such as alginic acid, Primogel (R) or corn starch; a lubricant such as magnesium stearate or Sterote; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1% of active compound, but may be varied between 0.5 and about 50% of the weight thereof. The amount of active compounds in such compositions is such that a suitable dosage will be obtained. Preferred compositions and preparations according to the present invention are prepared so that a parenteral dosage unit contains between 0.5 to 100 milligrams of active compound.

The solutions or suspensions may also include the following components: a sterile diluent such as water

for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

## EXAMPLE 1

### 4-(1H-Indazol-3-y1)piperidine-1-carboximidic acid methyl ester

To a stirred suspension of 1-benzoyl-3-(1-cyano-4-piperidinyl)-1H-indazole (7.9g, 0.024 mole) in methanol (15 ml) was added 4.9 ml of 25% solution of sodium methoxide in methanol. The solution was stirred at ambient temperature for 16 hours, made neutral to pH paper with glacial acetic acid, and extracted with ether. The aqueous solution was made basic with concentrated $NH_4OH$ , and extracted thrice with $CH_2Cl_2$ (35 ml) . The organic extracts were combined, washed ($H_2O$), dried ( $MgSO_4$) and the solvent removed in vacuo to yield 6.6 g of an oil. The oil was triturated with hexane and the resultant solid collected (5.4 g) . Recrystallization from $CH_3CN$ (twice) yielded 3.5 g (56.8%) of 4-(1H-indazol-3-yl)piperidine-1-carboximidic acid methyl ester having a melting point of 153-155° C.

ANALYSIS:
Calculated for $C_{14}H_{18}N_4O$ : 65.09%C 7.02%H 21.69%N
Found: 65.27%C 6.96%H 21.74%N

## EXAMPLE 2

### a. 4-(6-Fluoro-1H-indazol-3-yl)piperidine-1-carbonitrile

To a mixture of $NaHCO_3$ (2.3 g., 1.027 mol), BrCN (1,5 g, 0.0141 mol., 97%) and dimethyl sulfoxide (DMSO) [20 ml.] , was added dropwise 6-fluoro-3-(4-piperidinyl)-1H-indazole (3.0g., 0.014 mol) dissolved in 30 ml. of warm DMSO. After two hours at ambient temperature the reaction mixture was poured into water and the resultant solid was collected, dried and yielded 3.6 g. of 4-(6-fluoro-1H-indazol-3-yl)piperidine-1-carbonitrile, m.p. 143-145° C.

### b. 4- (6-fluoro-1H-indazol-3-yl) piperidine-1-carboximidic acid methyl ester

A solution of 4-(6-fluoro-1H-indazol-3-yl) piperidine-1-carbonitrile (3.0 g. 0.012 mol) of Example 2 a, methanol (35 ml) and 25% sodium methoxide in methanol (2.5 ml) was stirred at ambient temperature for 16 hours. Most of the methanol was removed in vacuo and the residue diluted with water. The aqueous mixture was extracted with $CH_2Cl_2$. The extract was washed ($H_2O$), dried ( $Na_2SO_4$) and the solvent evaporated to yield 3.0 g of a solid. The material was recrystallized from toluene to give 2.4 g (72%) of 4-(6-fluoro-1H-indazol-3-yl) piperidine-1-carboximidic acid methyl ester, m.p. 162-164° C.

ANALYSIS:
Calculated for $C_{14}H_{17}FN_4O$ : 60.85%C 6.20%H 20.28%N
Found: 6l.39%C 6.28%H 20.89%N

## EXAMPLE 3

4-(6-Fluoro-1-methyl-1H-indazol-3-yl) piperidine-1-carboximidic acid methyl ester

To a stirred mixture of cyanogen bromide (2.2 g, 0.021 mol) , sodium bicarbonate (3.5 g) and dimethyl sulfoxide (DMSO) (40 ml) was added 6-fluoro-1-methyl-3-(4-piperidinyl)-1H-indazole (5.0 g, 0.021 mol) in DMSO (50 ml) . The reaction was stirred at ambient temperature for 2 hours and then poured into water. The cyanamide separated from solution and was collected to yield 4.9 g of a solid. Recrystallization from ethylacetate-hexane yielded 3.6 g (66%) of 4-(6-fluoro-1-methyl-1H-indazol-3-yl) piperidine-1-carbonitrile,, mp. 133-136° C. To a stirred mixture of 4-(6-fluoro-1-methyl-1H-indazol-3-yl)-1-piperidine-1-carbonitrile (3.4 g, 0.013 mole) and methanol (40 ml) was added a 25% sodium methoxide-methanol solution (2.5 ml) . The mixture was warmed briefly to effect solution and was then stirred at ambient temperature for 16 hours. The methanol was concentrated and the residue was diluted with water. The resulting precipitate was collected to yield 3.8 g of the product as a solid. The material was recrystallized from toluene-hexane to yield 3.0 g of 4-(6-fluoro-1-methyl-1H-indazol-3-yl)piperidine-1-carboximidic acid methyl ester, mp 132-135° C.

ANALYSIS:
Calculated for $C_{15}H_{20}FN_4O$ 62.05%C 6.59%H 19.30%N
Found 61.95%C 6.54%H 19.13%N

EXAMPLE 4

4-(6-Chloro-3H-indazol-3-yl)piperidine-1-carboximidic acid methyl ester

To a stirred mixture of cyanogen bromide (1.8 g, 0.017 mol), sodium bicarbonate (2.80 g) and dimethyl sulfoxide (DMSO) (40 ml) was added dropwise 6-chloro-3-(4-piperidinyl)-1H-indazole (4.0 g, 0.017 mol) dissolved in DMSO (50 ml) . The reaction was stirred at ambient temperature for 1 hour and poured into water. The resulting solid was collected, dried and weighed 4.0 g. Recrystallization from toluene-hexane afforded 3.1 g (70%) of 4-(6-chloro-1H-indazol-3-yl)piperidine-1-carbonitrile, m.p. 180-188° C. To a stirred mixture of 4-(6-chloro-1H-indazol-3-yl)piperidine-1-carbonitrile (3.0 g., 0.0815 mol) in methanol (30 ml) was added a solution of 25% sodium methoxide in methanol (2.5 ml). The reaction was warmed on a steam bath to effect solution and then stirred at ambient temperature for 16 hours. Most of the methanol was removed in vacuo and the residue was diluted with water. An initial oil solidified and 3.5 g of a solid was collected. This was recrystallized from methanol-water to yield 2.9 g (66%) of 4-(6-chloro-1H-indazol-3-yl)piperidine-1-carboximidic acid methyl ester, mp 173-175° C.

ANALYSIS:
Calculated for $C_{14}H_{19}ClN_4O$ : 57.43%C 5.85%H 19.14%N
Found: 57.44%C 5.87%H 19.34%N

## Claims

**1.** A compound of the formula I

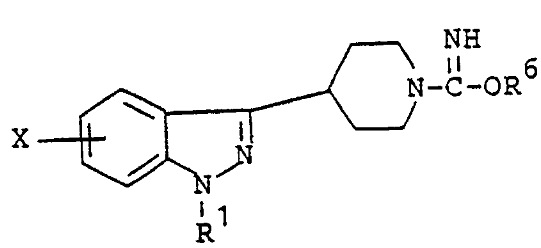

wherein

$R^1$ is hydrogen, $C_1$-$C_7$-alkyl, di-$C_1$-$C_7$-alkylamino-$C_1$-$C_7$-alkyl, hydroxy-$C_1$-$C_7$-alkyl, $C_3$-$C_7$-cycloalkyl-$C_1$-$C_7$-alkyl, a radical of the formula

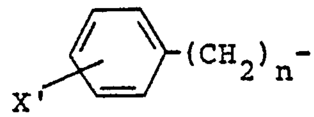

where n is an integer of 0 to 4 and X' is hydrogen, halogen or $CF_3$,

$R^6$ is $C_1$-$C_7$-alkyl, $C_3$-$C_7$-cycloalkyl-$C_1$-$C_7$-alkyl or $C_1$-$C_7$-alkyl substituted with amino, $C_1$-$C_7$-alkylamino or di-$C_1$-$C_7$-alkylamino,

X is hydrogen or halogen,

the optical antipodes or the pharmaceutically acceptable salts thereof.

2. The compound of claim 1 which is 4-(1H-indazol-3-yl)-piperidine-1-carboximidic acid methyl ester.

3. The compound of claim 1 which is 4-(6-fluoro-1H-indazol-3-yl)-piperidine-1-carboximidic acid methyl ester.

4. The compound of claim 1 which is 4-(6-fluoro-1-methyl-1H-indazol-3-yl)-piperidine-1-carboximidic acid methyl ester.

5. The compound of claim 1 which is 4-(6-chloro-1H-indazol-3-yl)-piperidine-1-carboximidic acid methyl ester.

6. A pharmaceutical composition comprising an effective amount of a compound as defined in claim 1 and an inert adjuvant.

7. Use of a compound as defined in claim 1 for the preparation of a medicament having blood pressure reducing activity.

8. A process for the preparation of a compound as defined in claim 1, which comprises reacting a compound of the formula II

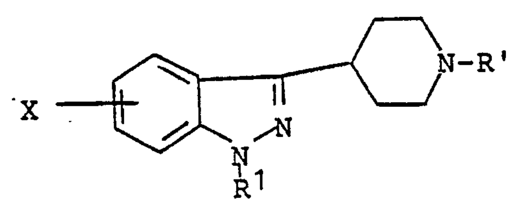

wherein $R^1$ and X are as defined above and R' is cyano, with an alcohol of the formula $R^6OH$ .

Claims for the Contracting State :AT

1. A process for the preparation of a compound of the formula I wherein

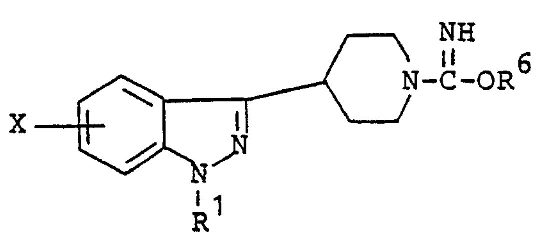

$R^1$ is hydrogen, $C_1$-$C_7$-alkyl, $C_3$-$C_7$-cycloalkyl-$C_1$-$C_7$-alkyl, di-$C_1$-$C_7$-alkylamino-$C_1$-$C_7$-alkyl hydroxy-$C_1$-$C_7$-alkyl, or a radical of the formula

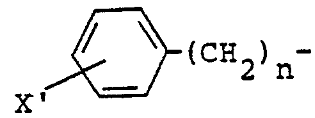

where n is an integer of 0 to 4 and X' is hydrogen, halogen or $CF_3$,

$R^6$ is $C_1$-$C_7$-alkyl, $C_3$-$C_7$-cycloalkyl-$C_1$-$C_7$-alkyl or $C_1$-$C_7$-alkyl substituted with amino, $C_1$-$C_7$-alkylamino or di-$C_1$-$C_7$-alkylamino,

X is hydrogen or halogen,

the optical antipode thereof; or the pharmaceutically acceptable salt thereof, which comprises reacting a compound of the formula II

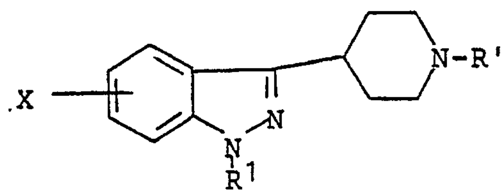

wherein $R^1$ and X are as defined above and R' is cyano, with an alcohol of the formula $R^6OH$ .

2. The process as defined in claim 1 wherein 4-(1H-indazol-3-yl)-piperidine-1-carboximidic acid methyl ester is prepared.

3. The process as defined in claim 1 wherein 4-(6-fluoro-1H-indazol-3-yl)-piperidine-1-carboximidic acid methyl ester is prepared.

4. The process as defined in claim 1 wherein 4-(6-fluoro-1-methyl-1H-indazol-3-yl)-piperidine-1-carboximidic acid methyl ester is prepared.

5. The process as defined in claim 1 wherein 4-(6-chloro-1H-indazol-3-yl)-piperidine-1-carboximidic acid methyl ester is prepared.

**Revendications**

**1.** Composé de formule 1

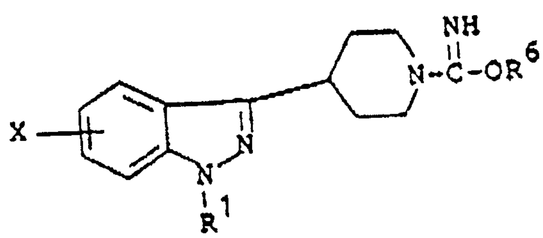

dans laquelle

$R^1$ est un atome d'hydrogène ou un radical alkyle en $C_1$-$C_7$, dialkyl ($C_1$-$C_7$)-amino-alkyle ($C_1$-$C_7$), hydroxyalkyle en $C_1$-$C_7$, cycloalkyl($C_3$-$C_7$)-alkyle($C_1$-$C_7$), ou un radical de formule

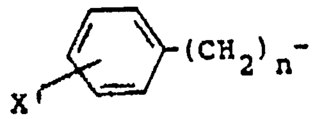

dans laquelle n est un nombre entier allant de 0 à 4 et X' est un atome d'hydrogène ou d'halogène ou $CF_3$,

R6 est un radical alkyle en $C_1$-$C_7$, cycloalkyl ($C_3$-$C_7$)-alkyle ($C_1$-$C_7$) ou un radical alkyle en $C_1$-$C_7$ substitué par un groupe amino, alkyl ($C_1$-$C_7$)-amino ou dialkyl ($C_1$-$C_7$)-amino,

X est un atome d'hydrogène ou d'halogène, et inverses optiques ou sels pharmaceutiquement acceptables de celui-ci.

2. Composé selon la revendication 1, qui est l'ester méthylique d'acide 4-(1H-indazol -3-yl)-pipéridine-1-carboxymidique.

3. composé selon la revendication 1, qui est l'ester méthylique d'acide 4-(6-fluoro-1H-indazol-3-yl)-pipéridine-1-carboxymidique.

4. Composé selon la revendication 1, qui est l'ester méthylique d'acide 4-(6-fluoro-1-méthyl-1H-indazol -3-yl)-pipéridine-1-carboxymidique.

5. Composé selon la revendication 1, lequel est l'ester méthylique d'acide 4-(6-chloro-1H-indazol -3-yl)-pipéridine-1-carboxymidique.

6. Composition pharmaceutique comprenant une quantité efficace d'un composé tel que défini dans la revendication 1, et un adjuvant inerte.

7. Utilisation d'un composé tel que défini dans la revendication 1, pour la fabrication d'un médicament ayant une activité de réduction de la pression artérielle.

8. Procédé pour la préparation d'un composé tel que défini dans la revendication 1, lequel comprend la mise en réaction d'un composé de formule II

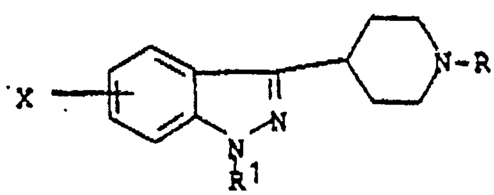

dans laquelle $R^1$ et X sont tels que définis plus haut et R' est le groupe cyano, avec un alcool de formule $R^6OH$ .

**Ansprüche**

Patentansprüche für den Vertragsstaat:AT

1. Verfahren zur Herstellung einer Verbindung der Formel I

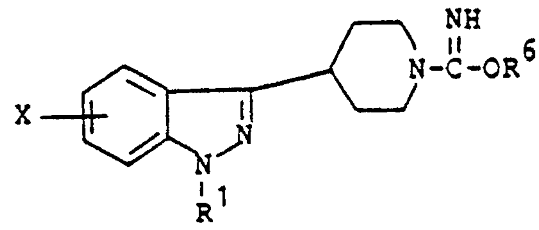

worin

$R^1$ für Wasserstoff, $C_1$-$C_7$-Alkyl, Di-$C_1$-$C_7$-alkylamino-$C_1$-$C_7$-alkyl, Hydroxy-$C_1$-$C_7$-alkyl, $C_3$-$C_7$-Cycloalkyl-$C_1$-$C_7$-alkyl oder einen Rest der Formel

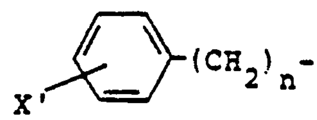

steht, worin n eine ganze Zahl von 0 bis 4 bedeutet und X' Wasserstoff, Halogen oder $CF_3$ darstellt,

$R^6$ für $C_1$-$C_7$-Alkyl, $C_3$-$C_7$-Cycloalkyl-$C_1$-$C_7$-alkyl oder $C_1$-$C_7$-Alkyl steht, welches mit Amino, $C_1$-$C_7$-Alkylamino oder Di-$C_1$-$C_7$-Alkyl-amino substituiert ist.

X Wasserstoff oder Halogen darstellt,

der optischen Antipoden hievon; oder der pharmazeutisch annehmbaren Salze hievon, welches das Umsetzen einer Verbindung der Formel II

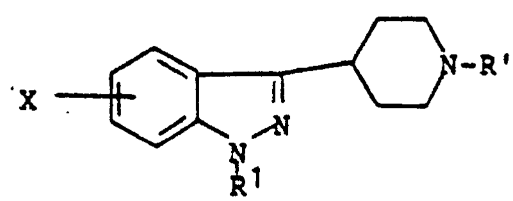

worin $R^1$ und X wie vorstehend definiert und R für Cyano steht, mit einem Alkohol der Formel $R^6OH$ umfaßt.

2. Verfahren nach Anspruch 1, worin 4-(1H-Indazol-3-yl)-piperidin-1-carboximidsäuremethylester hergestellt wird.

3. Verfahren nach Anspruch 1, worin 4-(6-Fluor-1H-indazol-3-yl))-piperidine-1-carboximidsäuremethylester hergestellt wird.

4. Verfahren nach Anspruch 1, worin 4-(6-Fluor-1-methyl-1H-indazol-3-yl)-piperidine-1-carboximidsäuremethylester hergestellt wird.

5. Verfahren nach Anspruch 1, worin 4-(6-chlor-1H-indazol-3-yl)-piperidine-1-carboximidsäuremethylester hergestellt wird.